# FASCICULE DE BREVET EUROPEEN

(11) **EP 2 303 345 B1**
(45) Date de publication et mention de la délivrance du brevet: **06.08.2014**
(21) Numéro de dépôt: 09784440.1
(22) Date de dépôt: 26.06.2009
(51) Int. Cl.: A61L 27/46

(54) **SUBSTITUT OSSEUX, ET PROCEDE POUR SA PREPARATION**
KNOCHENERSATZ UND VERFAHREN ZU SEINER HERSTELLUNG
BONE SUBSTITUTE, AND METHOD FOR THE PREPARATION THEREOF

(30) Priorité: 30.06.2008 FR 0803663
(43) Date de publication de la demande: 06.04.2011
(73) Titulaire: Université Pierre et Marie Curie (Paris 6), 75005 Paris (FR); Ecole Pratique des Hautes Etudes, 75007 Paris (FR); Centre National de la Recherche Scientifique, 75016 Paris (FR)
(72) Inventeur: NASSIF, Nadine, F-75005 Paris (FR); GOBEAUX, Frédéric, F-75013 Paris (FR); GIRAUD GUILLE, Marie, Madeleine, F-75012 Paris (FR); MOSSER, Gervaise, F-75014 Paris (FR)
(74) Mandataire: Goulard, Sophie
(86) Numéro de dépôt international: PCT/FR2009/051233
(87) Numéro de publication internationale: WO 2010/004182

(56) Documents cités:
- EP-A1- 1 566 186
- WO-A1-2007/055431
- TAMPIERI ANNA ET AL.: "Design of graded biomimetic osteochondral composite scaffolds" BIOMATERIALS, vol. 29, no. 26, 1 septembre 2008 (2008-09-01), pages 3539-3546, XP002514675 ELSEVIER SCIENCE PUBLISHERS BV., BARKING, GB ISSN: 0142-9612
- SACHLOS ELEFTERIOS ET AL.: "Collagen scaffolds reinforced with biomimetic composite nano-sized carbonate-substituted hydroxyapatite crystals and shaped by rapid prototyping to contain internal microchannels" TISSUE ENGINEERING, vol. 12, no. 9, septembre 2006 (2006-09), pages 2479-2487, XP002514644
- YOON B-H ET AL: "Stability and cellular responses to fluorapatite-collagen composites" BIOMATERIALS, ELSEVIER SCIENCE PUBLISHERS BV., BARKING, GB, vol. 26, no. 16, 1 juin 2005 (2005-06-01), pages 2957-2963, XP025280610 ISSN: 0142-9612 [extrait le 2005-06-01]
- KIKUCHI M ET AL: "Self-organization mechanism in a bone-like hydroxyapatite/collagen nanocomposite synthesized in vitro and its biological reaction in vivo" BIOMATERIALS, ELSEVIER SCIENCE PUBLISHERS BV., BARKING, GB, vol. 22, no. 13, 1 juillet 2001 (2001-07-01), pages 1705-1711, XP004245928 ISSN: 0142-9612
- W. ZHANG ET AL: 'Hierarchical Self-Assembly of Nano-Fibrils in Mineralized Collagen' CHEMISTRY OF MATERIALS vol. 15, no. 16, 01 Août 2003, pages 3221 - 3226, XP055066293 DOI: 10.1021/cm030080g ISSN: 0897-4756

## Description

La présente invention concerne un substitut osseux et un procédé pour sa préparation.

L'os est un matériau hybride constitué principalement par des cellules, du collagène de type I qui constitue une trame organique protéique et une phase minérale constituée de cristaux d'hydroxyapatite de taille nanométrique. Cette association organique/minéral à grande échelle en trois dimensions confère à la fois de l'élasticité et de la dureté au tissu osseux lui permettant de résister aux forces qui lui sont appliquées. L'os est donc dur, dense et très résistant.

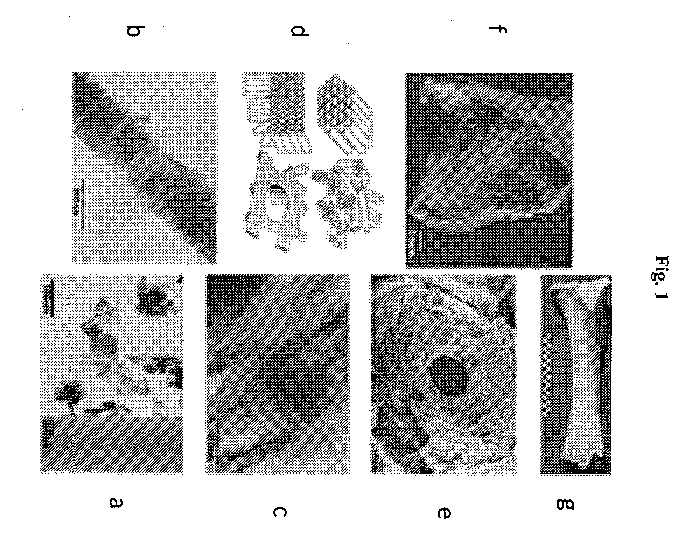

Il a été proposé par Weiner & Wagner (Ann. Rev. Mater. Sci. 28, 271-298, 1998) une description d'une organisation hiérarchisée à différentes échelles se décomposant en sept niveaux décrits comme suit et qui sont illustrés par la figure 1 annexée :
- Niveau 1 (Fig. la) : Les deux composants majoritaires de base constitutifs de l'os, c'est-à-dire les plaquettes d'hydroxyapatite et les fibrilles striées de collagène, constituent le premier niveau hiérarchique d'organisation. Il s'agit du plus bas niveau d'organisation, à l'échelle du nanomètre. La phase apatite est notamment caractérisée par la présence de plans inter réticulaires caractéristiques tels que (002) et (300). Les cristaux d'apatite, à ce niveau d'organisation, ne possèdent pas d'orientation particulière. Les fibrilles de collagène sont caractérisées par une striation périodique visible en microscopie électronique et résultant de l'assemblage des molécules de collagène I induisant un décalage périodique de 67 nm ;
- Niveau 2 (Fig. 1b) : Le coalignement des plaquettes d'hydroxyapatite selon leur axe c le long de l'axe principal des fibrilles striées de collagène constitue le second niveau, c'est-à-dire que les plans inter réticulaires (002) de l'apatite sont orientés perpendiculairement à l'axe principal des fibrilles et donc, selon la périodicité axiale (i.e. selon les striations) des fibrilles de collagène (striation = 67 nm). On parle de fibrilles minéralisées de collagène (largeur 100 à 300 manomètres). Le niveau 2 se situe également à l'échelle du nanomètre ;
- Niveau 3 (Fig. 1c) : Plusieurs fibrilles minéralisées de collagène sont assemblées côte à côte en faisceaux parallèles, formant une fibre minéralisée de collagène (largeur 1 à 3 micromètres). L'échelle du micromètre est atteinte au niveau 3 d'organisation ;
- Niveau 4 (Fig. 1 d) : II est complexe car les fibrilles ou bien les fibres minéralisées de collagène peuvent s'organiser en trois dimensions. En effet, on distingue à ce niveau la coexistence de domaines où les fibrilles/fibres sont alignées selon une direction préférentielle sur une grande distance et/ou forment des structures en arceaux caractéristiques de leur empilement selon une géométrie dite cholestérique. On distingue également des domaines où les fibrilles/fibres ne s'organisent pas (domaines dits isotropes). L'échelle varie du micro- au millimètre ;
- Niveau 5 (Fig. 1e) : L'os compact présente un agencement de structures cylindriques parallèles de taille millimétrique, désignées par "ostéones". En coupe, ces ostéones apparaissent constitués de lamelles concentriques de collagène ;
- Niveau 6 (Fig. 1f) : La partie centrale des os longs riche en cellules est appelée "os spongieux". Au niveau de cet os, des lamelles osseuses forment un réseau macroporeux de travées minces et irrégulières. L'association os compact/os spongieux constituent le niveau 6 d'organisation. On est au-delà du millimètre ;
- Niveau 7 (Fig. 1g) : Le dernier niveau est tout simplement l'os entier.

Plusieurs classes de matériaux synthétiques (désignés par "matériaux d'implants") ou naturels (désignés par "greffons") sont proposés dans l'art antérieur. Les matériaux d'implants sont généralement bio inertes, c'est-à-dire simplement tolérés par l'organisme, ou biocompatibles, c'est-à-dire qu'ils s'intègrent parfaitement à l'organisme hôte. Un greffon est un tissu osseux prélevé sur la personne à laquelle il est destiné (autogreffe) ou sur une tierce personne (allogreffe), et il est généralement ostéo-conducteur, c'est-à-dire qu'il est capable de guider la repousse osseuse.

Un substitut osseux ostéo-inducteur, c'est-à-dire capable d'induire une reconstruction osseuse, constitue néanmoins un substitut idéal. L'élaboration d'un tel matériau est complexe. Sa mise en place nécessite l'utilisation de constituants qui ont une nature chimique et une phase cristalline particulière afin d'optimiser sa parfaite intégration au sein d'un organisme humain ou animal, et d'éviter ainsi le rejet. Son organisation tridimensionnelle doit être reconstituée pour assurer d'une part les propriétés mécaniques et d'autre part une porosité appropriée à la colonisation dudit substitut par le tissu hôte. L'accès à l'organisation de la trame organique de l'os (20% en masse), ainsi que son association avec la phase minérale (70% en masse) dans le tissu sont très difficiles à reproduire *in vitro*.

De nombreux travaux ont été effectués en vue de la synthèse de substituts osseux, et notamment des travaux portant sur la minéralisation de collagène. La minéralisation de collagène de tendons osseux de dinde a été étudiée par W. Traub, et al. [Proc. Natl. Acad. Sci. USA 1989, 86, 9822-9826], mais le matériau obtenu ne présente pas une organisation analogue à celle de l'os. D'autres essais ont été effectués avec du collagène purifié *in vitro,* mais les conditions de forte dilution dans lesquelles les essais ont été effectués n'ont pas permis d'obtenir un matériau présentant la densité osseuse et l'organisation tridimensionnelle du collagène trouvées dans les tissus osseux vivants [Cf. D. Lickorish, et al. (J. Biomed. Mat. Res. 2004, 68A, 19-27); S. Yunoki, et al., (Mat. Lett., 2006, 60, 999-1002); D. A. Wahl, et al. (Eur. Cell. Mat. 2006, 11, 43-56)].

La cristallisation de calcite CaCO₃ à partir d'une solution de CaCl₂ sous une atmosphère d'ammoniac générée par la décomposition thermique à température ambiante d'une poudre de (NH₄)CO₃ a été décrite par L. Addadi, et al. (Proc. Natl. Acad. Sci. USA 1987, 84, 2732-2736).

Il est également connu de précipiter du collagène à partir d'une solution acide par une augmentation du pH. R. L. Ehrman, et al., (J. Nat. Cancer Inst. 1956, 16, 1375-1403) décrivent un procédé dans lequel une solution de collagène dans l'acide acétique est mise en contact avec des vapeurs de NH₃. Elle se transforme en un gel contenant des grains fins. La structure du matériau obtenu n'est pas décrite.

M.M. Giraud-Guille, et al. (J. Mol. Biol. 1995, 251, 197-202) et (J. Mol. Biol. 1992, 224, 861-873) décrivent la structure "cristal liquide" obtenue à partir d'une solution concentrée en collagène ainsi que la transition sol-gel obtenue par élévation du pH de acide à basique.

G. Mosser, M. M. Giraud-Guille, et al., (Matrix Biol. 2006, 25, 3-13) décrivent un procédé dans lequel une solution acide en collagène (5 mg/mL) est concentrée progressivement dans des microchambres en verre afin d'obtenir une organisation hélicoïdale à longue portée des molécules de collagène ainsi qu'un gradient de concentration. La solution est alors mise en présence de vapeurs d'ammoniac, pour former des fibrilles de collagène et stabiliser l'organisation mise en place en phase liquide.

B. A. Harley, et al., (Biomaterials 2006, 27, 866-874) décrivent l'élaboration d'une matrice structurée de collagène contenant également un glucosaminoglycane. Des microfibrilles de collagène sont mélangées au sulfate de chondroïtine de manière homogène à 4°C. La solution est ensuite centrifugée dans un moule, congelée de manière ultra rapide, lyophilisée, puis réticulée à 105°C sous un vide de 50 mTorr pendant 24 heures. La nature fibrillaire du collagène n'est pas décrite.

C. Guo, et al., (Biomaterials 2007, 28, 1105-1114) décrivent l'utilisation de billes magnétiques pour aligner une solution de fibrilles de collagène. Une solution de collagène préparée dans un tampon phosphate à des concentrations de 2,5 mg/ml, maintenue à 4°C, est mise en présence des billes magnétiques. Les mêmes échantillons sont également préparés en présence de cellules à une concentration finale en collagène à 1,2 mg/ml. Dans les deux cas, les échantillons sont mis dans un champ magnétique de moins de 1G lors de l'induction de la fibrillogenèse produite par une élévation de température à 37°C. Une atmosphère en CO₂ est également utilisée lorsque des cellules sont intégrées dans la matrice. Les matrices sont très lâches et la nature fibrillée du collagène n'est pas mentionnée. M. J. Olsza, et al., (Calcif. Tissue Int. 2003, 72, 583-591) décrivent la calcification d'une éponge de collagène, en présence ou en absence d'un polymère du type poly(acide aspartique). L'éponge de collagène est constituée par du collagène de type I obtenu à partir de tendon bovin. Le minéral est du carbonate de calcium et non du phosphate de calcium, aucune phase apatite n'est donc obtenue. La présence de fibres striées n'est pas démontrée et les fibres de collagène ne sont pas orientées. J. H. Bradt, et al., (Chem. Mater. 1999, 11, 2694-2701**)** décrivent un procédé dans lequel on prépare à 4°C, d'une part une solution de collagène (collagène de type I de derme de veau) à 1 mg/mL acidifiée par HCl et contenant CaCl₂, et d'autre part une solution tampon contenant des ions phosphate. La solution de phosphate est ensuite mélangée à la solution de collagène permettant d'atteindre un pH de 6,8, et l'ensemble est chauffé à 30°C. La coprécipitation donne un mélange de phases contenant du phosphate de calcium, de l'hydroxyapatite et du phosphate octacalcique. De plus, les fibres de collagène sont isolées non orientées et ne constituent pas une matrice dense. N. Gehrke, N. Nassif, et al., (Chem. Mater. 2005, 17, 6514-6516) décrivent la reminéralisation par du carbonate de calcium, en présence ou en l'absence d'un polymère du type poly(acide aspartique), de la trame organique de la nacre préalablement déminéralisée.

W. Zhang, et al. (Chemistry of materials 2003, 15, 3221-3226) décrit la préparation de nano-fibrilles de collagène minéralisé pour former des structures mimant l'os naturel. Une solution de collagène à une concentration de 0,6 mg/mL est mélangée à une solution de NaH₂PO₄ pour former un matériau composite présentant des cristaux d'apatite co-alignés avec les fibrilles de collagène.

Aucun des procédés de synthèse connus à ce jour ne permet d'obtenir un substitut osseux reproduisant le niveau 4 d'organisation tridimensionnelle du collagène associé à une phase minérale de cristaux d'apatite que l'on observe dans l'os naturel.

Le but de la présente invention est de fournir un matériau synthétique utilisable comme substitut osseux biocompatible, ayant une structure très proche de la structure de l'os vivant (niveau 4), ainsi qu'un procédé pour sa préparation.

Le matériau synthétique selon la présente invention comprend une phase organique (I) et une phase minérale (II).

La phase organique (I) comprend des fibrilles striées de collagène constituées par des triples hélices de collagène I et dans lesquelles la périodicité des striations est d'environ 67 nm, lesdites fibrilles étant ordonnées à grande distance selon une géométrie 3D associant des domaines alignés et des domaines cholestériques, ainsi que des domaines isotropiques où elles ne sont pas ordonnées.

La phase minérale (II) comprend des cristaux d'apatite de structure cristalline hexagonale, groupe d'espace 6/m, lesdits cristaux comprenant au moins des ions calcium et au moins des ions phosphate.

Dans le matériau conforme à l'invention, l'axe c des cristaux d'apatite de la phase minérale est co-aligné avec l'axe longitudinal des fibrilles striées de collagène de la phase organique.

La teneur en collagène au sein dudit matériau est d'au moins 75 mg/cm³.

Au sein dudit matériau, l'ordre de grandeur des différents domaines (cholestérique, alignement, isotrope) est d'une cinquantaine de microns environ.

Selon une forme de réalisation particulière, la phase minérale est constituée de cristaux d'hydroxyapatite pure. Au sens de la présente invention, on entend par « hydroxyapatite pure », une hydroxyapatite exempte d'autres phases de phosphate cristallisées telle que la brushite.

Dans un mode de réalisation particulier de l'invention, la phase minérale est constituée de cristaux d'hydroxyapatite stoechiométrique de formule (I) suivante :

Ca₁₀(PO₄)₆(OH)₂ (I)

Selon une forme de réalisation particulière de l'invention, le rapport atomique Ca/P des cristaux d'hydroxyapatite de formule (I) est de 1,67.

Selon une autre forme de réalisation de l'invention, la phase minérale comprend des cristaux d'apatite comprenant en outre au moins des ions hydroxyde et dans lesquels les ions phosphate (type B) et/ou les ions hydroxyde (type A) sont partiellement remplacés par des ions carbonates.

Dans ces hydroxyapatites, un ou plusieurs sites de la structure cristalline peut(peuvent) être exempt(s) d'ion(s). Il s'agit dans ce cas d'hydroxyapatites non stoechiométriques comportant ce que l'on appelle alors une ou plusieurs lacunes ioniques.

Dans un autre mode de réalisation, la phase minérale comprend des cristaux d'apatite comprenant des ions Ca²⁺, des ions PO₄³⁻ et des ions OH⁻, et dans laquelle au moins l'un des ions Ca²⁺, PO₄³⁻ ou OH⁻ est partiellement remplacé par d'autres ions.

Parmi les ions susceptibles de remplacer partiellement les ions Ca²⁺, on peut citer les ions Mg²⁺,Cu²⁺, Sr²⁺, Ba²⁺, Zn²⁺, Cd²⁺, Pb²⁺, Na⁺, K⁺ et Eu³⁺.

Parmi les ions susceptibles de remplacer partiellement les ions PO₄³⁻, on peut mentionner les ions CO₃²⁻, SiO₄³⁻, AsO₄³⁻, MnO₄³⁻, VO₄³⁻, CrO₄³⁻ et HPO₄²⁻. 2-Parmi les ions susceptibles de remplacer partiellement les ions OH-, on peut mentionner les ions CO₃²⁻, F⁻, Cl⁻, Br⁻, I⁻, S²⁻ et O²⁻.

Le matériau selon l'invention peut en outre contenir une quantité infinitésimale de protéoglycanes, de glycosaminoglycanes et/ou de molécules organiques favorisant la minéralisation. Par quantité infinitésimale, on entend une proportion inférieure à 2%.

Les caractéristiques d'un matériau de l'invention peuvent être déterminées par des analyses en microscopie optique, des analyses en microscopie électronique à balayage MEB, des analyses en microscopie électronique à transmission MET, et des analyses en diffraction des rayons X.

Des lames semifines d'un matériau selon l'invention, observées en microscopie optique en lumière polarisée montrent des propriétés de biréfringence. Dans un cas idéal, l'observation d'alternances de bandes éclairées et de bandes éteintes associée au déplacement de ces franges lors de la rotation de la platine du microscope, indique une structure hélicoïdale.

Des échantillons du matériau de l'invention, analysés en MEB, montrent des fibrilles orientées immergées dans une gangue minéralisée, sans amas cristallins individualisés d'ordre micrométrique.

Des clichés de diffusion des rayons X aux petits angles, effectués sur un matériau de l'invention, montrent le signal anisotrope des fibrilles de collagène et les harmoniques de la période D = 67 nm. Des clichés de diffusion des rayons X aux grands angles montrent les pics principaux de la phase apatite. L'existence d'un coalignement entre le signal des fibrilles et celui du minéral caractérise le matériau de l'invention. Ce coalignement est mis plus particulièrement en évidence localement dans les zones où les fibrilles sont alignées.

Un matériau selon l'invention peut être obtenu par un procédé consistant à préparer une solution aqueuse acide initiale de collagène précurseur de la phase organique (I), et au moins une solution aqueuse de précurseurs de la phase minérale (II) et à faire précipiter le collagène par élévation du pH jusqu'à une valeur d'au moins 7. Il est caractérisé en ce que :
- la concentration en collagène au sein de la solution aqueuse acide est d'au moins 75 mg/ml et reste constante durant ladite élévation de pH,
- les précurseurs de la phase minérale comprennent au moins un sel de calcium et au moins un sel de phosphate,
- la précipitation de la phase minérale (II) est effectuée par mise en contact de la solution de précurseurs de la phase minérale avec la phase organique (I), ladite mise en contact étant effectuée avant ou après la précipitation de ladite phase organique (I). La durée du contact est fixée selon la vitesse de précipitation et le taux de charge minérale envisagé dans le matériau final.

Dans un premier mode de mise en oeuvre du procédé, on fait précipiter le collagène de la phase organique (I) avant sa mise en contact avec une solution neutre de précurseurs de la phase minérale (II). Dans ce cas, la solution de précurseurs de la phase minérale (II) contient au moins lesdits sels de calcium et au moins lesdits sels de phosphate. La proportion de phase minérale dans le matériau final est modulée par la quantité d'ions apportée à la solution. Dans ce mode de mise en oeuvre, le collagène a acquis sa structure fibrillaire avant sa mise en contact avec la phase minérale.

Dans un deuxième mode de mise en oeuvre du procédé, une solution acide de précurseurs de la phase organique (I) est mise en contact avec une solution acide de précurseurs de (II). Le mélange est alors soumis à une augmentation de pH qui induit la coprécipitation du collagène et de l'apatite. Dans ce mode de mise en oeuvre, le maintien constant de la concentration initiale en collagène est particulièrement important. Un premier moyen pour éviter la dilution est de contenir la solution acide concentrée de précurseurs de la phase organique (I) dans un moule, dont la forme est adaptée à l'usage recherché et enfermé dans une membrane de dialyse. Un second moyen est d'introduire la solution concentrée de collagène dans une enveloppe souple constituée par une membrane de dialyse. Le moule ou ladite enveloppe sont ensuite immergés dans la solution de précurseurs de la phase minérale (II).

Lorsque la phase minérale II du matériau conforme à l'invention est constituée de cristaux d'hydroxyapatite pure, le procédé est de préférence mis en oeuvre dans une enceinte close dans laquelle on dispose :
- au moins un premier récipient contenant une solution aqueuse d'au moins un sel de phosphate et d'au moins un sel de calcium, précurseurs de la phase minérale II, dans laquelle est plongé au moins un boudin de dialyse contenant une solution aqueuse acide initiale de collagène précurseur de la phase organique (I),
- au moins un deuxième récipient contenant une solution d'ammoniaque ou une poudre de (NH₄)₂CO₃ ;
étant entendu que :
- le rapport (volume de la solution de précurseurs de la phase minérale I)/(volume interne de l'enceinte close) est de 2.10⁻³ environ,
- la hauteur de la solution de précurseurs de la phase minérale I contenue dans le premier récipient varie de 3 à 5 cm environ, le diamètre dudit récipient étant de 2 à 5 cm environ ;
- le rapport (volume de la solution aqueuse d'ammoniaque)/(volume interne de l'enceinte close) est de 8.10⁻³ ou le rapport volume de la poudre de (NH₄)₂CO₃ /(volume interne de l'enceinte close) est de 6.10⁻³ environ.

Le respect de ces conditions conduit à l'obtention d'un matériau dans lequel la phase minérale II est constituée d'apatite pure, exempte de tout autre type de phase de phosphate de calcium.

La solution aqueuse acide initiale de collagène présente de préférence les caractéristiques suivantes :
- sa concentration en collagène est entre 75 mg/mL et 1000 mg/mL, de préférence entre 100 mg/mL et 400 mg/mL
- son pH est inférieur à 4, de préférence inférieur à 3, en présence d'acides, de préférence d'acide acétique 0,5 M.

La solution de précurseurs de la phase minérale (II) présente de préférence les caractéristiques suivantes :
- la concentration en précurseur de calcium, par exemple CaCl₂, est inférieure à la limite de solubilité, de préférence de 2,5 mM à 1,5 M, plus particulièrement de 11 à 550 mM) ;
- la concentration en précurseur de phosphate, par exemple NaH₂PO₄, est inférieure à la limite de solubilité, de préférence de 1,5 à 900 mM, plus particulièrement de 66 à 330 mM ;
- les quantités de précurseurs sont telles que le rapport molaire Ca/P est entre 1,5 et 1,8, de préférence de l'ordre de 1,67.

A titre d'exemple, la limite de solubilité à 20°C est de 7,08 M pour NaH₂PO₄, et de 3,83 M pour CaCl₂.

Lorsque la phase minérale du matériau recherché comprend des cristaux d'apatite comprenant des ions Ca²⁺, des ions PO₄³⁻ et des ions OH⁻, et dans laquelle au moins l'un des ions Ca²⁺, PO₄³⁻ ou OH⁻ est partiellement remplacé par d'autres ions, la solution de précurseurs de la phase minérale contient en outre un ou plusieurs sels dont le cation est destiné à remplacer au moins partiellement Ca²⁺, et/ou un ou plusieurs sels dont l'anion est destiné à remplacer au moins partiellement PO₄³⁻ et/ou OH⁻.

Les sels des cations destinés à remplacer Ca²⁺ sont avantageusement choisis parmi les sels contenant des cations monovalents ou divalents, tels que par exemple MgCl₂, BaCl₂, SrCl₂, NaCl, KCl, et NH₄Cl. Le précurseur de CO₃²⁻ peut être NaHCO₃. La quantité de précurseur de CO₃²⁻ est de préférence telle que le rapport NaH₂PO₄/NaHCO₃ soit égal à 1. En présence de carbonate, le rapport molaire Ca/(P+C) est entre 1,5 et 1,8, de préférence de l'ordre de 1,67.

La solution de précurseurs de la phase minérale (II) peut contenir en outre des protéoglycanes, des glycosaminoglycanes et/ou des molécules organiques favorisant la minéralisation, telles que des chaînes de polymères d'acides aminés acides, de préférence un poly(acide aspartique) ayant une longueur de chaîne entre 5 et 150 unités acide aminé, de préférence environ 15, et avec une concentration entre 0,01 µg/mL et 1,5 mg/mL, de préférence 10 µg/mL.

L'élévation du pH est effectuée avantageusement par une atmosphère gazeuse basique, en particulier une atmosphère de NH₃, ou de (NH₄)₂CO₃ dans un mode de réalisation particulier dans lequel PO₄³⁻ ou OH⁻ et partiellement remplacé par CO₃²⁻.

Dans un mode de réalisation particulier, le procédé comprend une étape supplémentaire au cours de laquelle on imprègne le matériau obtenu par coprécipitation, par une solution dite SBF ("Simulated Body Fluid") analogue à un fluide biologique, puis on ajuste le pH du milieu à 7,4.

| | |
|---|---|
| NaCl | de 137 à 213 mM (par exemple 213,0 mM) |
| NaHCO₃ | de 1,2 à 6,3 mM (par exemple 6,3 mM) |
| KCl | de 3 à 4,5 mM (par exemple 4,5 mM) |
| K₂HPO₄•3H₂O | de 1 à 1,5 mM (par exemple 1,5 mM) |
| CaCl₂ | de 2,6 à 3,8 mM (par exemple 3,8 mM) |
| Na₂SO₃Na₂SO₄ | de 0,5 à 0,75 mM (par exemple 0,75 mM) |
| MgCl₂•6H₂O | de 1,5 à 2,3 mM (par exemple 2,3 mM). |

Les concentrations de cette solution SBF représentent environ 1,5 fois celles effectivement mesurées pour un fluide biologique (Cf. Zhang L.-J., et al., Mater. Lett. 2004, 58, 719-722).

Le pH peut être ajusté à 7,4 avec un mélange de tris(hydroxyméthyl)aminométhane 0,01 mol/L et d'HCl à 0,01 mol/L à 37°C.

La présente invention est décrite plus en détail à l'aide des exemples suivants, auxquels elle n'est cependant pas limitée.

Dans les exemples, on a utilisé un collagène de type I préparé à partir de queues de jeunes rats Wistar, selon le mode opératoire suivant. Les tendons de queue de rat sont excisés dans une hotte à flux laminaire stérile, puis lavés dans une solution tampon phosphate saline contenant 137 mM de NaCl, 2,68 mM de KCl, 8,07 mM de Na₂HPO₄, et 1,47 mM de NaH₂PO₄, pour éliminer les cellules et les traces de sang. Les tendons sont ensuite trempés dans une solution de NaCl 4 M pour éliminer les cellules intactes restantes et précipiter une partie des protéines à poids moléculaire élevé. Après un nouveau lavage avec la solution tampon saline, les tendons sont dissous dans une solution aqueuse à 500 mM d'acide acétique. La solution ainsi obtenue est clarifiée par centrifugation à 41000 g pendant 2 h. Les protéines autres que le collagène de type I sont précipitées de manière sélective dans une solution aqueuse de NaCl 300 mM, puis éliminées par centrifugation à 41000 g pendant 3 h. Le collagène est récupéré à partir du surnageant par précipitation dans une solution aqueuse de NaCl 600 mM suivie d'une centrifugation à 3000g pendant 45 min. Les culots ainsi obtenus sont dissous dans une solution aqueuse d'acide acétique 500 mM, puis dialysés soigneusement dans le même solvant pour éliminer totalement NaCl.

Les solutions sont maintenues à 4°C et centrifugées à 41000 g pendant 4 h avant d'être utilisées. Des solutions de collagène à différentes concentrations sont préparées par dialyse inverse contre du polyéthylène glycol (35 kDa, Fluka) dissous dans une solution aqueuse d'acide acétique 500 mM, jusqu'à 50% (m/v) ou par évaporation lente dans une hotte à flux laminaire. La concentration en collagène de la solution acide a été déterminée avant la fibrillogenèse par détermination de la quantité d'hydroxyproline. Bien entendu, d'autres sources de collagène peuvent être utilisées.

### Exemple 1

On a préparé une solution précurseur de la phase minérale en dissolvant dans 40 mL d'eau, 110 mM de NaH₂PO₄, 66 mM de CaCl₂, 500 mM d'acide acétique et 0,40 µg de poly(acide aspartique). La solution est équilibrée à pH 2,2.

La solution de collagène utilisée dans cet exemple a été dosée à environ 300 mg/mL. Elle se présente sous forme d'un gel élastique partiellement fibrillaire.

La solution de collagène a été introduite dans un boudin de dialyse (PM = 3500 Da), et le boudin a été placé dans la solution de précurseurs de la phase inorganique dans un récipient ouvert. Ledit récipient a ensuite été placé sous atmosphère d'ammoniac jusqu'à complète précipitation des sels à une température de 20°C.

L'atmosphère d'ammoniac a provoqué une coprécipitation de collagène et d'hydroxyapatite, visible dès 3 heures. On peut laisser le milieu réactionnel mûrir pendant 8 jours.

Les échantillons ont été lavés par immersion dans une solution, avantageusement une solution tampon au phosphate PBS (phosphate buffer solution).

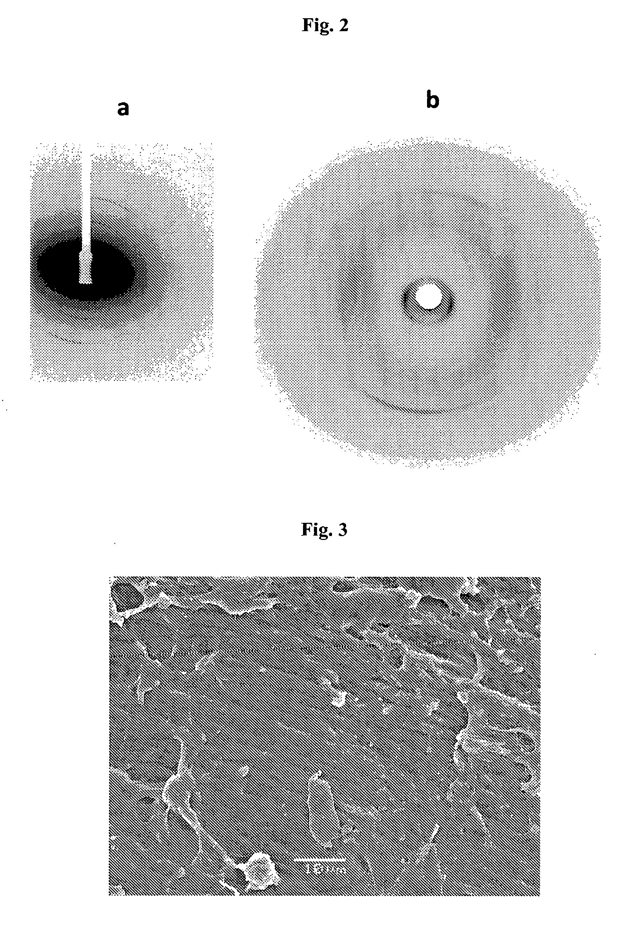

La figure 2 illustre l'analyse du matériau par diffusion des rayons X. La figure 2a représente un cliché SAXS et la figure 2b représente un cliché WAXS. Le cliché SAXS montre le signal anisotrope des fibrilles de collagène et les harmoniques de la période D = 67 nm ; ceci met en évidence la périodicité des striations tous les 67 nm le long de l'axe principal des fibrilles. Le cliché WAXS montre que la réflexion (002), caractéristique de la présence d'apatite est renforcée dans la même direction que le signal des fibrilles observé en (a). Ceci indique donc que l'axe *c* des cristaux d'apatite est orienté le long de l'axe principal des fibrilles de collagène. Le signal correspondant à l'interdistance d_{latéral} des molécules de collagène dans la fibrille est perpendiculaire à la réflexion (002) et parallèle à la réflexion (300) de l'apatite. Les plans inter réticulaire sont donc orientés préférentiellement selon la direction des molécules de collagène. Cette signature en diffraction des rayons X est comparable à celle trouvée sur l'os.

La figure 3 représente une micrographie MEB. Elle montre la présence de fibrilles orientées (domaines alignés) minéralisées.

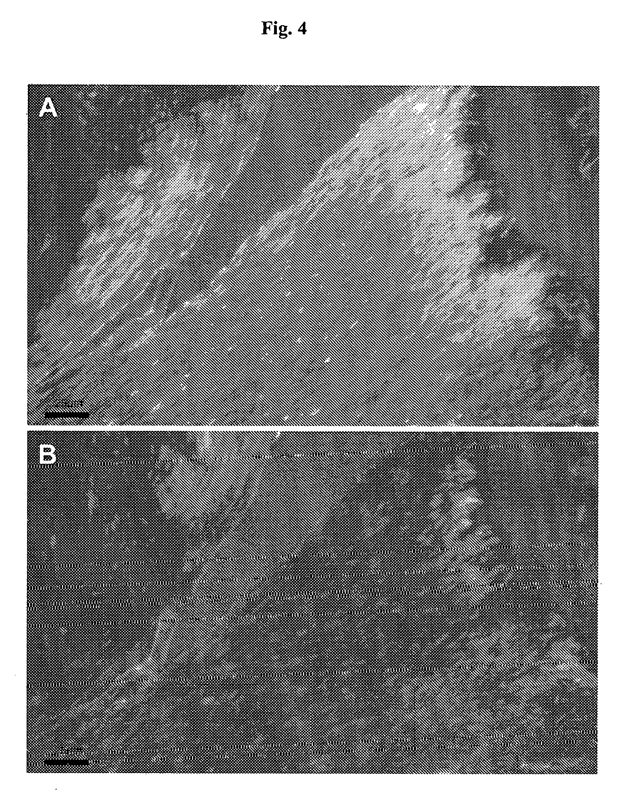

La figure 4 représente une coupe semi-fine colorée au bleu de toluidine qui sert d'agent contrastant du matériau, observé par microscopie optique à lumière polarisée entre polariseurs croisés (A,B). 4B représente la même zone que 4A, également observée entre polariseurs croisés mais tournés de 45° par rapport à 4A. La biréfringence est due d'une part à l'organisation de la phase organique et d'autre part à son imprégnation par la phase minérale. La variation des bandes de biréfringence entre les deux positions des polariseurs indique la coexistence de domaines distincts : (i) un domaine aligné caractérisé par une zone de biréfringence qui s'éteint entre 4A et 4B car les fibrilles de collagène minéralisées sont alignées les unes par rapport aux autres ; et (ii) un domaine cholestérique caractérisée par une zone de biréfringence dont l'alternance de bandes claires et sombres s'inversent entre 4A et 4B car l'orientation des fibrilles de collagène minéralisées tourne régulièrement d'un plan à l'autre. Un troisième domaine coexiste avec les deux précédents, il s'agit d'un domaine dit « isotrope » où les fibrilles de collagène minéralisées se répartissent de façon aléatoire au sein du matériau ; ce domaine ne présente donc aucune zone de biréfringence en 4A et 4B.

Des mesures des propriétés mécaniques du matériau ainsi préparé ont également été effectuées, en particulier le module élastique, selon la technique de nanoindentation. Les mesures ont été effectuées avec un système nanomécanique d'indentation de type Ubi 1 (Hysitron Inc., Minneapolis, MN, USA) et une pointe d'indentation Berkovich, ~10 µm². Il a été trouvé que le rapport des modules élastiques à 0° et 90° par rapport à l'axe longitudinal des fibrilles de collagène est de 1,43 ± 1,18. L'ordre de grandeur de ce rapport est comparable à celui obtenu pour un os compact natif soit 1,50 ± 0,315, indiquant que le degré d'anisotropie du présent matériau et ainsi son organisation fibrillaire est similaire à celui trouvé dans l'os.

### Exemple 2

Une solution diluée de collagène (1 mg/L) a été injectée de manière à contrecarrer une évaporation d'eau dans une microchambre en verre de 15 uL. L'injection s'est poursuivie jusqu'à obtenir une phase dense cristalline liquide de collagène. Le collagène a été précipité sous atmosphère d'ammoniac, puis la microchambre a été immergée dans la solution de précurseurs minéraux (ladite solution contenant: NaCl 213,0 mM, NaHCO₃ 6,3 mM, KCl 4,5 mM, K₂HPO₄•3H₂O 1,5 mM, CaCl₂ 3,8 mM, Na₂SO₃Na₂SO₄ 0,75 mM, et MgCl₂•6H₂O 2,3 mM) ajustée pH à 7,4 et maintenue dans cette solution pendant une durée de 6 mois à une température de 37°C.

Ensuite, le matériau précipité a été lavé par immersion dans une solution tampon au phosphate (PBS).

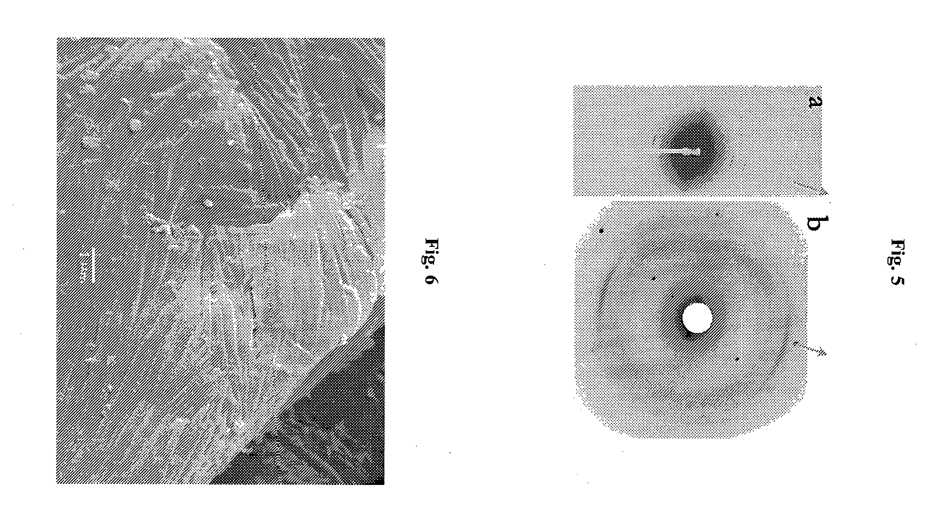

La figure 5 illustre l'analyse du matériau par diffusion des rayons X. 5a représente un cliché SAXS et 5b représente un cliché WAXS. Les deux clichés sont analogues à ceux de l'exemple 1.

### Exemple 3

Une solution diluée à 5 mg/mL de collagène, préalablement dialysée contre une solution de NaH₂PO₄ (66 mM) et de CaCl₂ (110 mM), a été injectée de manière à contrecarrer une évaporation d'eau dans une microchambre en verre de 15 µL. L'injection s'est poursuivie jusqu'à obtenir une phase dense cristalline liquide de collagène. La microchambre a été immergée dans une solution de précurseurs de la phase inorganique analogue à celle de l'exemple 1, dans un récipient ouvert. Ledit récipient a ensuite été placé sous atmosphère de carbonate d'ammonium jusqu'à complète précipitation des sels à une température de 20°C.

L'atmosphère d'ammoniac et de dioxyde de carbone a provoqué une coprécipitation de collagène et d'hydroxyapatite, visible dès 3 heures. On peut laisser le milieu réactionnel mûrir pendant 8 jours.

Les échantillons ont été lavés par immersion dans une solution tampon au phosphate PBS (PBS).

La figure 6 représente une micrographie MEB. Elle montre la présence de fibres minéralisées présentant une organisation hélicoïdale (domaine cholestérique).

## Revendications

1. Matériau synthétique comprenant une phase organique (I) et une phase minérale (II), **caractérisé en ce que** :
- la phase organique (I) comprend des fibrilles striées de collagène constituées par des triples hélices de collagène I et dans lesquelles la périodicité des striations est de 67 nm, lesdites fibrilles étant ordonnées à grande distance selon une géométrie 3D associant des domaines alignés et des domaines cholestériques, ainsi que des domaines isotropiques où elles ne sont pas ordonnées ;
- la phase minérale (II) comprend des cristaux d'apatite de structure cristalline hexagonale, groupe d'espace 6/m, lesdits cristaux comprenant au moins des ions calcium et au moins des ions phosphate ;
- l'axe c desdits cristaux d'apatite de la phase minérale est co-aligné avec l'axe longitudinal des fibrilles striées de collagène de la phase organique ;
- la teneur en collagène au sein dudit matériau est d'au moins 75 mg/cm³.

2. Matériau selon la revendication 1, **caractérisé en ce que** la phase minérale est constituée de cristaux d'hydroxyapatite pure.

3. Matériau selon la revendication 1 ou 2, **caractérisé en ce que** la phase minérale est constituée de cristaux d'hydroxyapatite stoechiométrique de formule (I) suivante :
Ca₁₀(PO₄)₆(OH)₂ (I)

4. Matériau selon la revendication 3, **caractérisé en ce que** le rapport atomique Ca/P des cristaux d'hydroxyapatite de formule (I) est de 1,67.

5. Matériau selon la revendication 1, **caractérisé en ce que** la phase minérale comprend des cristaux d'apatite comprenant en outre au moins des ions hydroxyde et dans lesquels les ions phosphate et/ou les ions hydroxyde sont partiellement remplacés par des ions carbonates.

6. Matériau selon la revendication 1, **caractérisé en ce que** la phase minérale comprend des cristaux d'apatite comprenant des ions Ca²⁺, des ions PO₄³et des ions OH⁻, et dans laquelle l'un au moins des ions Ca²⁺, PO₄³⁻ ou OH⁻ est partiellement remplacé par d'autres ions, étant entendu que :
- les ions susceptibles de remplacer partiellement les ions Ca²⁺ sont choisis parmi les ions Mg²⁺,Cu²⁺, Sr²⁺, Ba²⁺, Zn²⁺, Cd²⁺, Pb²⁺, Na⁺, K⁺ et Eu³⁺ ;
- les ions susceptibles de remplacer partiellement les ions PO₄³⁻ sont choisis parmi les ions CO₃²⁻, SiO₄³⁻, AsO₄³⁻, MnO₄³⁻, VO₄³⁻, CrO₄³⁻ et HPO₄²⁻ ; et
- les ions susceptibles de remplacer partiellement les ions OH-, sont choisis parmi les ions CO₃²⁻, F⁻, Cl⁻, Br⁻, I⁻, S²⁻ et O²⁻.

7. Matériau selon l'une quelconque des revendications précédentes, **caractérisé en ce qu'**il contient en outre une quantité de protéoglycanes, de glycosaminoglycanes et/ou de molécules organiques favorisant la minéralisation, inférieure à 2%.

8. Procédé de préparation d'un matériau selon la revendication 1, consistant à préparer une solution aqueuse acide initiale de collagène précurseur de la phase organique (I), et au moins une solution aqueuse de précurseurs de la phase minérale (II) et à faire précipiter le collagène par élévation du pH jusqu'à une valeur d'au moins 7, **caractérisé en ce que** :
- la concentration en collagène au sein de la solution aqueuse acide est d'au moins 75 mg / ml et reste constante durant ladite élévation du pH,
- les précurseurs de la phase minérale comprennent au moins un sel de calcium et au moins un sel de phosphate,
- la précipitation de la phase minérale (II) est effectuée par mise en contact de la solution de précurseurs de la phase minérale avec la phase organique (I), ladite mise en contact étant effectuée avant ou après la précipitation de ladite phase organique (I).

9. Procédé selon la revendication 8, **caractérisé en ce que** l'on fait précipiter le collagène de la phase organique (I) avant sa mise en contact avec une solution neutre de précurseurs de la phase minérale (II), ladite solution neutre contenant au moins lesdits sels de calcium et au moins lesdits sels de phosphate.

10. Procédé selon la revendication 8, **caractérisé en ce qu'**une solution acide de précurseurs de la phase organique (I) est mise en contact avec une solution acide de précurseurs de la phase minérale (II), et le mélange ainsi obtenu est soumis à une augmentation de pH qui induit la coprécipitation du collagène et de l'apatite.

11. Procédé selon la revendication 10, **caractérisé en ce que** la solution acide concentrée de précurseurs de la phase organique (I) est contenue dans un moule qui est enfermé dans une membrane de dialyse, l'ensemble étant ensuite immergé dans la solution de précurseurs de la phase minérale (II).

12. Procédé selon la revendication 10, **caractérisé en ce que** la solution concentrée de collagène est introduite dans une enveloppe souple constituée par une membrane de dialyse, ladite enveloppe étant ensuite immergée dans la solution de précurseurs de la phase minérale (II).

13. Procédé selon la revendication 8, pour la préparation d'un matériau dans lequel la phase minérale II est constituée de cristaux d'hydroxyapatite pure et tel que défini à la revendication 2, **caractérisé en ce qu'**il est mis en oeuvre dans une enceinte close dans laquelle on dispose :
- au moins un premier récipient contenant une solution aqueuse d'au moins un sel de phosphate et d'au moins un sel de calcium, précurseurs de la phase minérale II, dans laquelle est plongé au moins un boudin de dialyse contenant une solution aqueuse acide initiale de collagène précurseur de la phase organique (I),
- au moins un deuxième récipient contenant une solution d'ammoniaque ou une poudre de (NH₄)₂CO₃ ;
étant entendu que :
- le rapport (volume de la solution de précurseurs de la phase minérale I) / (volume interne de l'enceinte close) est de 2.10⁻³,
- la hauteur de la solution de précurseurs de la phase minérale I contenue dans le premier récipient varie de 3 à 5 cm, le diamètre dudit récipient étant de 2 à 5 cm environ ;
- le rapport (volume de la solution aqueuse d'ammoniaque)/(volume interne de l'enceinte close) est de 8.10⁻³ ou le rapport volume de la poudre de (NH₄)₂CO₃ /(volume interne de l'enceinte close) est de 6.10⁻³.

14. Procédé selon la revendication 8, **caractérisé en ce que** la solution aqueuse acide initiale de collagène présente les caractéristiques suivantes :
- sa concentration en collagène est de 75 mg/mL à 1000 mg/mL ;
- son pH est inférieur à 4, en présence d'acides.

15. Procédé selon la revendication 8, **caractérisé en ce que** la solution de précurseurs de la phase minérale (II) présente les caractéristiques suivantes :
- la concentration en précurseur de calcium est inférieure à la limite de solubilité ;
- la concentration en précurseur de phosphate est inférieure à la limite de solubilité ;
- les quantités de précurseurs sont telles que le rapport molaire Ca/P est entre 1,5 et 1,8.

16. Procédé selon la revendication 8, pour la préparation d'un matériau selon la revendication 6, **caractérisé en ce que** la solution de précurseurs de la phase minérale contient en outre un ou plusieurs sels dont le cation est destiné à remplacer au moins partiellement Ca²⁺, et/ou un ou plusieurs sels dont l'anion est destiné à remplacer au moins partiellement PO₄³⁻ ou OH⁻.

17. Procédé selon la revendication 8, pour la préparation d'un matériau selon la revendication 7, **caractérisé en ce que** la solution de précurseurs de la phase minérale (II) contient en outre des protéoglycanes, des glycosaminoglycanes et/ou des molécules organiques favorisant la minéralisation.

18. Procédé selon la revendication 8, **caractérisé en ce que** l'élévation du pH est effectuée par une atmosphère gazeuse basique.

## Patentansprüche

1. Synthetisches Material, das eine organische Phase (I) und eine mineralische Phase (II) umfasst, **dadurch gekennzeichnet, dass**:
- die organische Phase (I) gestreifte Kollagenfibrillen umfasst, die von Kollagen-I-Dreifachhelix gebildet werden und in denen die Periodizität der Streifungen 67 nm beträgt, wobei die Fibrillen in großem Abstand gemäß einer 3D-Geometrie, die alignierte Domänen und cholesterische Domänen kombiniert, geordnet sind, sowie isotropische Domänen, wo sie nicht geordnet sind,
- die mineralische Phase (II) Apatitkristalle mit hexagonaler Kristallstruktur umfasst, Raumgruppe 6/m, wobei die Kristalle mindestens Kalziumionen und mindestens Phosphationen umfassen,
- die Achse c der Apatitkristalle der mineralischen Phase mit der Längsachse der gestreiften Kollagenfibrillen der organischen Phase coaligniert ist,
- der Kollagengehalt innerhalb des Materials mindestens 75 mg/cm³ beträgt.

2. Material nach Anspruch 1, **dadurch gekennzeichnet, dass** die mineralische Phase aus Kristallen reinen Hydroxyapatits besteht.

3. Material nach Anspruch 1 oder 2, **dadurch gekennzeichnet, dass** die mineralische Phase aus Kristallen stöchiometrischen Hydroxyapatits der folgenden Formel (I) besteht:
Ca₁₀(PO₄)₆(OH)₂ (I).

4. Material nach Anspruch 3, **dadurch gekennzeichnet, dass** das Atomverhältnis Ca/P der Hydroxyapatitkristalle der Formel (I) 1,67 ist.

5. Material nach Anspruch 1, **dadurch gekennzeichnet, dass** die mineralische Phase Apatitkristalle umfasst, die ferner mindestens Hydroxidionen umfassen und in denen die Phosphationen und/oder die Hydroxidionen teilweise durch Carbonationen ersetzt sind.

6. Material nach Anspruch 1, **dadurch gekennzeichnet, dass** die mineralische Phase Apatitkristalle umfasst, die Ca²⁺-Ionen, PO₄³⁻-Ionen und OH⁻-Ionen umfassen und in der mindestens eines der Ca²⁺-, PO₄³⁻- oder OH⁻Ionen teilweise durch andere Ionen ersetzt ist, wobei davon ausgegangen wird, dass:
- die Ionen, die imstande sind, die Ca²⁺-Ionen teilweise zu ersetzen, aus den Ionen Mg²⁺,Cu²⁺, Sr²⁺, Ba²⁺, Zn²⁺, Cd²⁺, Pb²⁺, Na⁺, K⁺ und Eu³⁺ ausgewählt sind,
- die Ionen, die imstande sind, die PO₄³⁻-Ionen teilweise zu ersetzen, aus den Ionen CO₃²⁻, SiO₄³⁻, AsO₄³⁻, MnO₄³⁻, VO₄³⁻, CrO₄³⁻ und HPO₄²⁻ ausgewählt sind, und
- die Ionen, die imstande sind, die OH--Ionen teilweise zu ersetzen, aus den Ionen CO₃²⁻, F⁻, CI⁻, Br⁻, I⁻, S²⁻ und O²⁻ ausgewählt sind.

7. Material nach einem der vorangehenden Ansprüche, **dadurch gekennzeichnet, dass** es ferner eine Menge von Proteoglycanen, Glycosaminoglycanen und/oder von organischen Molekülen, die die Mineralisierung fördern, von unter 2 % enthält.

8. Verfahren zur Herstellung eines Materials nach Anspruch 1, das darin besteht, eine saure wässrige Kollagenvorläufer-Ausgangslösung der organischen Phase (I) herzustellen und mindestens eine wässrige Vorläuferlösung der mineralischen Phase (II) und das Kollagen durch Hebung des pH bis auf einen Wert von mindestens 7 auszufällen, **dadurch gekennzeichnet, dass**:
- die Kollagenkonzentration innerhalb der sauren wässrigen Lösung mindestens 75 mg/ml beträgt und während der pH-Hebung konstant bleibt,
- die Vorläufer der mineralischen Phase mindestens ein Kalziumsalz und mindestens ein Phosphatsalz umfassen,
- die Ausfällung der mineralischen Phase (II) durch Inkontaktversetzen der Vorläuferlösung der mineralischen Phase mit der organischen Phase (I) erfolgt, wobei das Inkontaktversetzen vor oder nach dem Ausfällen der organischen Phase (I) erfolgt.

9. Verfahren nach Anspruch 8, **dadurch gekennzeichnet, dass** die Ausfällung des Kollagens der organischen Phase (I) vor seinem Inkontaktversetzen mit einer neutralen Vorläuferlösung der mineralischen Phase (II) durchgeführt wird, wobei die neutrale Lösung mindestens die Kalziumsalze und mindestens die Phosphatsalze enthält.

10. Verfahren nach Anspruch 8, **dadurch gekennzeichnet, dass** eine saure Vorläuferlösung der organischen Phase (I) mit einer sauren Vorläuferlösung der mineralischen Phase (II) in Kontakt versetzt wird und das derart gewonnene Gemisch einer pH-Erhöhung unterzogen wird, die die Co-Ausfällung des Kollagens und des Apatits induziert.

11. Verfahren nach Anspruch 10, **dadurch gekennzeichnet, dass** die saure Lösung, in der die Vorläufer der organischen Phase (I) konzentriert sind, in einer Form enthalten ist, die in einer Dialysemembran eingeschlossen ist, wobei die Gruppe danach in die Vorläuferlösung der mineralischen Phase (II) eingetaucht wird.

12. Verfahren nach Anspruch 10, **dadurch gekennzeichnet, dass** die konzentrierte Kollagenlösung in eine elastische Hülle eingeführt wird, die von einer Dialysemembran gebildet wird, wobei die Hülle dann in die Vorläuferlösung der mineralischen Phase (II) eingetaucht wird.

13. Verfahren nach Anspruch 8 für die Herstellung eines Materials, bei dem die mineralische Phase II von Kristallen reinen Hydroxyapatits gebildet wird und wie in Anspruch 2 definiert, **dadurch gekennzeichnet, dass** es in einen geschlossenen Behälter umgesetzt wird, in dem zur Verfügung stehen:
- mindestens ein erster Behälter, der eine wässrige Lösung mindestens eines Phosphatsalzes und mindestens eines Kalziumsalzes als Vorläufer der mineralischen Phase II enthält, in die mindestens ein Dialysestrang getaucht wird, der eine saure wässrige Kollagenvorläufer-Ausgangslösung der organischen Phase (I) enthält,
- mindestens ein zweiter Behälter, der eine Ammoniaklösung oder ein (NH₄)₂CO₃-Pulver enthält,
wobei davon ausgegangen wird, dass:
- das Verhältnis (Volumen der Vorläuferlösung der mineralischen Phase I) / (internes Volumen des geschlossenen Behälters) 2,10⁻³ beträgt,
- die Höhe der Vorläuferlösung der mineralischen Phase I, die im ersten Behälter enthalten ist, von 3 bis 5 cm schwankt, wobei der Durchmesser des Behälters zirka 2 bis 5 cm beträgt,
- das Verhältnis (Volumen der wässrigen Ammoniaklösung) / (internes Volumen des geschlossenen Behälters) 8,10⁻³ beträgt oder das Verhältnis Volumen des (NH₄)₂CO₃-Pulvers / (internes Volumen des geschlossenen Behälters) 6,10⁻³ beträgt.

14. Verfahren nach Anspruch 8, **dadurch gekennzeichnet, dass** die saure wässrige Kollagen-Ausgangslösung die folgenden Merkmale aufweist:
- ihre Kollagenkonzentration beträgt von 75 mg/mL bis 1000 mg/mL,
- ihr pH ist kleiner als 4, bei der Anwesenheit von Säuren.

15. Verfahren nach Anspruch 8, **dadurch gekennzeichnet, dass** die Vorläuferlösung der mineralischen Phase (II) die folgenden Merkmale aufweist:
- die Kalziumvorläuferkonzentration liegt unter der Löslichkeitsgrenze,
- die Phosphatvorläuferkonzentration liegt unter der Löslichkeitsgrenze,
- die Vorläufermengen sind derart, dass das molare Verhältnis Ca/P zwischen 1,5 und 1,8 ist.

16. Verfahren nach Anspruch 8 für die Herstellung eines Materials nach Anspruch 6, **dadurch gekennzeichnet, dass** die Vorläuferlösung der mineralischen Phase ferner ein oder mehrere Salze enthält, deren Kation dazu bestimmt ist, Ca²⁺ mindestens teilweise zu ersetzen, und/oder ein oder mehrere Salze, deren Anion dazu bestimmt ist, PO₄³⁻ oder OH- mindestens teilweise zu ersetzen.

17. Verfahren nach Anspruch 8 für die Herstellung eines Materials nach Anspruch 7, **dadurch gekennzeichnet, dass** die Vorläuferlösung der mineralischen Phase (II) ferner Proteoglycane, Glycosaminoglycane und/oder organische Moleküle enthält, die die Mineralisierung fördern.

18. Verfahren nach Anspruch 8, **dadurch gekennzeichnet, dass** die Hebung des pH durch eine basische gasförmige Atmosphäre durchgeführt wird.

## Claims

1. A synthetic material comprising an organic phase (I) and a mineral phase (II), **characterized in that**:
- the organic phase (I) comprises striated collagen fibrils constituted of collagen I triple helices and in which the periodicity of the striations is 67 nm, said fibrils being organized over a large distance according to a 3D geometry associating aligned domains and cholesteric domains, and also isotropic domains where they are not organized;
- the mineral phase (II) comprises apatite crystals having a hexagonal crystalline structure, space group 6/m, said crystals comprising at least calcium ions and at least phosphate ions;
- the axis *c* of the apatite crystals of the mineral phase is coaligned with the longitudinal axis of the striated collagen fibrils of the organic phase;
- the collagen content in said material is at least 75 mg/cm³.

2. The material as claimed in claim 1, **characterized in that** the mineral phase consists of crystals of pure hydroxyapatite.

3. The material as claimed in claim 1 or 2, **characterized in that** the mineral phase consists of crystals of stoichiometric hydroxyapatite of formula (I) below:
Ca₁₀(PO₄)₆(OH)₂ (I)

4. The material as claimed in claim 3, **characterized in that** the Ca/P atomic ratio of the crystals of hydroxyapatite of formula (I) is 1.67.

5. The material as claimed in claim 1, **characterized in that** the mineral phase comprises apatite crystals also comprising at least hydroxide ions and in which the phosphate ions and/or the hydroxide ions are partially replaced with carbonate ions.

6. The material as claimed in claim 1, **characterized in that** the mineral phase comprises crystals of apatite comprising Ca²⁺ ions, PO₄³⁻ ions and OH⁻ ions, and in which at least one of the Ca²⁺, PO₄³⁻ or OH⁻ ions is partially replaced with other ions, it being understood that:
- the ions capable of partially replacing the Ca²⁺ ions are chosen from Mg²⁺,Cu²⁺, Sr²⁺, Ba²⁺, Zn²⁺, Cd²⁺, Pb²⁺, Na⁺, K⁺ and Eu³⁺ ions;
- the ions capable of partially replacing the PO₄³⁻ ions are chosen from CO₃²⁻, SiO₄³⁻, AsO₄³⁻, MnO₄³⁻, VO₄³⁻, CrO₄³⁻ and HPO₄²⁻ ions; and
- the ions capable of partially replacing the OH⁻ ions are chosen from CO₃²⁻, F⁻, Cl⁻, Br⁻, I⁻, S²⁻ and O²⁻ ions.

7. The material as claimed in any one of the preceding claims, **characterized in that** it also contains an amount of proteoglycans, of glycosaminoglycans and/or of organic molecules which promote mineralization, of less than 2%.

8. A method for preparing a material as claimed in claim 1, which consists in preparing an initial acidic aqueous solution of collagen which is a precursor for the organic phase (I), and at least one aqueous solution of precursors for the mineral phase (II), and in precipitating the collagen by increasing the pH to a value of at least 7, **characterized in that**:
- the concentration of collagen in the acidic aqueous solution is at least 75 mg/ml and remains constant during said increase in pH,
- the mineral phase precursors comprise at least one calcium salt and at least one phosphate salt,
- the precipitation of the mineral phase (II) is carried out by bringing the mineral phase precursor solution into contact with the organic phase (I), said bringing into contact being carried out before or after the precipitation of said organic phase (I).

9. The method as claimed in claim 8, **characterized in that** the collagen of the organic phase (I) is precipitated before it is brought into contact with a neutral solution of precursors for the mineral phase (II), said neutral solution containing at least said calcium salts and at least said phosphate salts.

10. The method as claimed in claim 8, **characterized in that** an acidic solution of precursors for the organic phase (I) is brought into contact with an acidic solution of precursors for the mineral phase (II), and the resulting mixture is subjected to an increase in pH which induces coprecipitation of the collagen and the apatite.

11. The method as claimed in claim 10, **characterized in that** the concentrated acidic solution of precursors for the organic phase (I) is contained in a mold which is enclosed in a dialysis membrane, the whole then being immersed in the solution of precursors for the mineral phase (II).

12. The method as claimed in claim 10, **characterized in that** the concentrated solution of collagen is introduced into a flexible envelope constituted of a dialysis membrane, said envelope then being immersed in the solution of precursors for the mineral phase (II).

13. The method as claimed in claim 8, for preparing a material in which the mineral phase II consists of crystals of pure hydroxyapatite and as defined in claim 2, **characterized in that** it is carried out in a closed chamber in which are placed:
- at least one first container containing an aqueous solution of at least one phosphate salt and at least one calcium salt, which are precursors for the mineral phase II, in which at least one dialysis bag is immersed, said dialysis bag containing an initial acidic aqueous solution of collagen which is a precursor for the organic phase (I),
- at least one second container containing an aqueous ammonia solution or an (NH₄)₂CO₃ powder;
it being understood that:
- the (volume of mineral phase I precursor solution)/(closed chamber internal volume) ratio is 2 × 10⁻³,
- the height of the mineral phase I precursor solution contained in the first container ranges from 3 to 5 cm, the diameter of said container being from 2 to 5 cm approximately;
- the (volume of aqueous ammonia solution)/(closed chamber internal volume) ratio is 8 × 10⁻³ or the (volume of (NH₄)₂CO₃ powder)/(closed chamber internal volume) ratio is 6 × 10⁻³.

14. The method as claimed in claim 8, **characterized in that** the initial acidic aqueous solution of collagen has the following characteristics:
- its collagen concentration is from 75 mg/mL to 1000 mg/mL;
- its pH is less than 4, in the presence of acids.

15. The method as claimed in claim 8, **characterized in that** the solution of precursors for the mineral phase (II) has the following characteristics:
- the concentration of calcium precursor is less than the solubility limit;
- the concentration of phosphate precursor is less than the solubility limit;
- the amounts of precursors are such that the Ca/P molar ratio is between 1.5 and 1.8.

16. The method as claimed in claim 8, for preparing a material as claimed in claim 6, **characterized in that** the solution of precursors for the mineral phase also contains one or more salts, the cation of which is intended to at least partly replace Ca²⁺, and/or one or more salts, the anion of which is intended to at least partially replace PO₄³⁻ or OH⁻.

17. The method as claimed in claim 8, for preparing a material as claimed in claim 7, **characterized in that** the solution of precursors for the mineral phase (II) also contains proteoglycans, glycosaminoglycans and/or organic molecules which promote mineralization.

18. The method as claimed in claim 8, **characterized in that** the increase in the pH is carried out by means of a basic gaseous atmosphere.
